# EUROPEAN PATENT SPECIFICATION

(11) **EP 4 464 320 B1**
(45) Date of publication and mention of the grant of the patent: **24.06.2026**
(21) Application number: 23740067.6
(22) Date of filing: 12.01.2023
(51) Int. Cl.: A61K 9/19, A61K 31/473, A61K 45/06, A61K 47/12, A61P 1/08

(54) **PHARMACEUTICAL COMPOSITION COMPRISING NEUROKININ-1 ANTAGONIST PRODRUG COMPOUND**
PHARMAZEUTISCHE ZUSAMMENSETZUNG MIT NEUROKININ-1-ANTAGONIST-PRODRUG-VERBINDUNG
COMPOSITION PHARMACEUTIQUE COMPRENANT UN COMPOSÉ DE PROMÉDICAMENT ANTAGONISTE DE LA NEUROKININE-1

(30) Priority: 12.01.2022 CN 202210032508
(43) Date of publication of application: 20.11.2024
(73) Proprietor: Jiangsu Hengrui Pharmaceuticals Co., Ltd., Lianyungang, Jiangsu 222047 (CN)
(72) Inventor: JU, Mingzhu, Lianyungang, Jiangsu 222047 (CN); WANG, Xiaoyan, Lianyungang, Jiangsu 222047 (CN); WANG, Jie, Lianyungang, Jiangsu 222047 (CN); CAO, Xiaoli, Lianyungang, Jiangsu 222047 (CN)
(74) Representative: dompatent
(86) International application number: PCT/CN2023/071901
(87) International publication number: WO 2023/134725

(56) References cited:
- WO-A1-2011/019911
- WO-A1-2020/259675
- WO-A1-2020/259675
- CN-A- 102 573 475
- CN-A- 111 388 657
- US-A1- 2019 358 249
- US-A1- 2020 188 368

## Description

### TECHNICAL FIELD

The present disclosure belongs to the field of pharmaceutical preparations, and in particular relates to a pharmaceutical composition comprising a neurokinin-1 antagonist prodrug compound and a preparation method therefor.

### BACKGROUND

Tachykinins are peptide ligands for neurokinin receptors. Neurokinin receptors, such as NK1, NK2 and NK3, are involved in various biological processes. They can be found in the nervous and circulatory systems of mammals, as well as in surrounding tissues. Therefore, the regulation of such receptors has been investigated for potential treatment or prevention of various physiological disorders, conditions or diseases in mammals.

On the other hand, drug-induced hemolysis is caused by the destruction of a large number of red blood cells due to immune factors after drugs enter the human body, and clinical signs of hemolysis include anemia, jaundice and soy sauce-colored urine. Drug-induced hemolytic anemia can be divided into the following three types: (1) drug-induced immune hemolytic anemia, which results in an antibody-mediated hemolytic reaction; (2) hemolytic anemia caused by the action of drugs on red blood cells with an inherited enzyme deficiency (e.g., G6PD deficiency); (3) hemolytic anemia caused by a drug-induced hemolytic reaction to abnormal hemoglobin. The key to treating this disease is to discontinue the use of related drugs and control the occurrence of hemolysis so as to prevent complications.

WO 2020259675 provides a new NK1 antagonist prodrug compound (formula I) that is effective in the treatment of various physiological disorders, conditions or diseases with minimal side effects, and that has good pharmaceutical activity.

WO 2011/019911 relates to pharmaceutical compositions of a neurokinin-1 antagonist (formula 1) and a pharmaceutically acceptable vehicle for intravenous administration to a patient in need of treatment

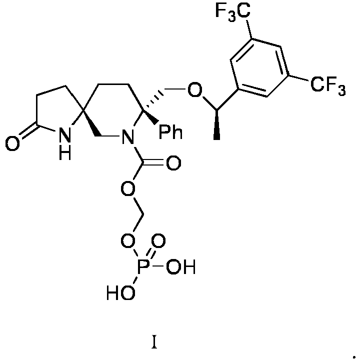

### CONTENT OF THE PRESENT INVENTION

An objective of the present disclosure is to provide a pharmaceutical composition comprising a compound of formula I or a pharmaceutically acceptable salt thereof, which composition has excellent stability.

An aspect of the present disclosure provides a pharmaceutical composition comprising a compound of formula I or a pharmaceutically acceptable salt thereof and a stabilizer, wherein the stabilizer is an amino acid and/or a saccharide,

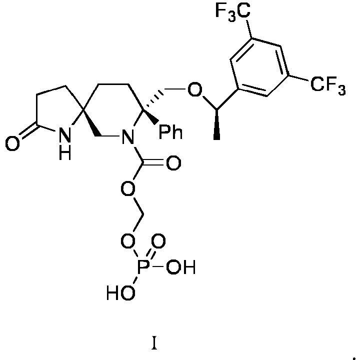

In some embodiments, the amino acid is an amino acid that contains at least one carboxylic acid group [-C(=O)OH] and at least one primary amino group or secondary amino group [-NH₂ or -RNH, wherein -R is a group other than -H] but does not contain a secondary amide group [-C(=O)-NH-]. For example, the amino acid may be one or more of alanine, 4-aminobutyric acid, 3-aminopentanoic acid, 5-aminopentanoic acid, 6-aminohexanoic acid, 8-aminooctanoic acid, arginine, aspartic acid, asparagine, cysteine, glutamic acid, glutamine, glycine, histidine, isoleucine, leucine, lysine, methionine, methyllysine, ornithine, phenylalanine, proline, serine, threonine, tryptophan, tyrosine and valine.

In some embodiments, the amino acid is a basic amino acid, preferably one or more of arginine, lysine and histidine, and more preferably arginine.

In some embodiments, the saccharide is selected from one or more of sucrose, glucose, lactose, trehalose and maltose, preferably sucrose and/or glucose, and more preferably glucose.

In some embodiments, the stabilizer is selected from one or more of arginine, glucose and sucrose, more preferably arginine and/or glucose.

In some embodiments, the pharmaceutical composition further comprises a solvent. Available solvents include, but are not limited to, water, organic solvents, water/organic solvent cosolvent systems. The organic solvents include, but are not limited to, methanol, ethanol, n-propanol, isopropanol, n-butanol, isobutanol, tert-butanol, acetone, acetonitrile, dichloromethane, dimethyl sulfoxide, etc. Preferred solvents may be water, ethanol, isopropanol, tert-butanol, water/ethanol, water/isopropanol, water/tert-butanol, etc.

In some embodiments, the weight ratio of the compound of formula I or the pharmaceutically acceptable salt thereof to the stabilizer is 1 : 0.1-1 : 20, e.g., 1 : 0.1, 1 : 0.2, 1 : 0.3, 1 : 0.4, 1 : 0.5, 1 : 0.6, 1 : 0.7, 1 : 0.8, 1 : 0.9, 1 : 1, 1 : 2, 1 : 3, 1 : 4, 1 : 5, 1 : 6, 1 : 7, 1 : 8, 1 : 9, 1 : 10, 1 : 11, 1 : 12, 1 : 13, 1 : 14, 1 : 15, 1 : 16, 1 : 17, 1 : 18, 1 : 19, 1 : 20 or any value therebetween, preferably 1 : 1-1 : 10, and more preferably 1 : 1-1 : 7.5.

In some embodiments, the compound of formula I or the pharmaceutically acceptable salt thereof has a mass volume percentage of 0.01%-30%, e.g., 0.01%, 0.05%, 0.1%, 0.2%, 0.5%, 1%, 2%, 3%, 4%, 5%, 6%, 7%, 8%, 9%, 10%, 12%, 14%, 16%, 18%, 20%, 22%, 24%, 26%, 28%, 30% or any value therebetween, preferably 0.01%-10%, and more preferably 0.1%-5%.

In some embodiments, the stabilizer has a mass volume percentage of 0.01%-30%, e.g., 0.01%, 0.05%, 0.1%, 0.2%, 0.5%, 1%, 2%, 3%, 4%, 5%, 6%, 7%, 8%, 9%, 10%, 12%, 14%, 16%, 18%, 20%, 22%, 24%, 26%, 28%, 30% or any value therebetween, preferably 0.01%-20%, and more preferably 0.1%-10%.

In some embodiments, the pharmaceutical composition further comprises a buffer. The buffer may be a phosphate buffer, a citrate buffer, an acetate buffer, a succinate buffer, a histidine salt buffer or other organic acid salt buffers.

In some embodiments, the buffer has a concentration of 5 mM-40 mM, preferably 10 mM-30 mM.

In some embodiments, the pharmaceutical composition may further comprise a pH regulator. The pH regulator includes, but is not limited to, sodium hydroxide, hydrochloric acid, etc.

In some embodiments, the pharmaceutical composition further comprises a chelating agent. The chelating agent is selected from ethylenediamine tetraacetic acid or a salt thereof.

In some embodiments, the weight ratio of the compound of formula I or the pharmaceutically acceptable salt thereof to the chelating agent is 1000 : 1-1 : 1, e.g., 1000 : 1, 900 : 1, 800 : 1, 700 : 1, 600 : 1, 500 : 1, 400 : 1, 300 : 1, 200 : 1, 100 : 1, 90 : 1, 80 : 1, 70 : 1, 60 : 1, 50 : 1, 45 : 1, 40 : 1, 35 : 1, 30 : 1, 25 : 1, 20 : 1, 15 : 1, 10 : 1, 9 : 1, 8 : 1, 7 : 1, 6 : 1, 5 : 1, 4 : 1, 3 : 1, 2 : 1, 1 : 1 or any value therebetween, more preferably 1000 : 1-10 : 1. In some embodiments, the chelating agent has a mass volume percentage of 0.001%-5%, preferably 0.01%-2%.

In some embodiments, the pharmaceutical composition has a pH value of 3.0-8.0, e.g., 3.0, 3.5, 4.0, 4.5, 5.0, 5.5, 6.0, 6.5, 7.0, 7.5, 8.0 or any value therebetween, preferably 5.0-8.0.

In some embodiments, the pharmaceutical composition further comprises a 5-HT3 receptor antagonist selected from granisetron, ondansetron, ramosetron, tropisetron, palonosetron or dolasetron, preferably palonosetron.

In some embodiments, the weight ratio of the compound of formula I or the pharmaceutically acceptable salt thereof to the 5-HT3 receptor antagonist is 2000 : 1-10 : 1, e.g., 2000 : 1, 1900 : 1, 1800 : 1, 1700 : 1, 1600 : 1, 1500 : 1, 1400 : 1, 1300 : 1, 1200 : 1, 1100 : 1, 1000 : 1, 900 : 1, 800 : 1, 700 : 1, 600 : 1, 500 : 1, 400 : 1, 300 : 1, 200 : 1, 100 : 1, 90 : 1, 80 : 1, 70 : 1, 60 : 1, 50 : 1, 45 : 1, 40 : 1, 35 : 1, 30 : 1, 25 : 1, 20 : 1, 15 : 1, 10 : 1 or any value therebetween, preferably 1000 : 1-100 : 1. In some embodiments, the 5-HT3 receptor antagonist has a mass volume percentage of 0.0001%-1%, e.g., 0.0001%, 0.001%, 0.01%, 0.05%, 0.1%, 0.2%, 0.3%, 0.4%, 0.5%, 0.6%, 0.7%, 0.8%, 0.9%, 1% or any value therebetween, preferably 0.0001%-0.1%.

In some embodiments, the pharmaceutical composition comprises (based on mass volume percentage):
0.01%-10% of a compound of formula I or a pharmaceutically acceptable salt thereof;
0.01%-20% of a stabilizer selected from one or more of arginine, glucose and sucrose;
optionally 0.0001%-1% of palonosetron or a pharmaceutically acceptable salt thereof;
optionally a 5 mM-40 mM buffer;
optionally 0.01%-2% of a chelating agent; and
70%-99% of a solvent selected from water and/or tert-butanol.

The pharmaceutical composition may have a pH value of 3.0-8.0, preferably 5.0-8.0. The pharmaceutical composition may optionally further comprise a pH regulator, etc.

The present disclosure further provides a sterile powder obtained by freeze-drying or spray-drying the pharmaceutical composition of the present disclosure.

The present disclosure further provides a sterile powder, which is reconstituted to obtain the pharmaceutical composition of the present disclosure.

In some embodiments, the sterile powder of the present disclosure contains less than 5% of prototype drug impurities, and the content thereof may be less than 4.9%, 4.8%, 4.7%, 4.6%, 4.5%, 4.4%, 4.3%, 4.2%, 4.1%, 4.0%, 3.9%, 3.8%, 3.7%, 3.6%, 3.5%, 3.4%, 3.3%, 3.2%, 3.1%, 3.0%, 2.9%, 2.8%, 2.7%, 2.6%, 2.5%, 2.4%, 2.3%, 2.2%, 2.1%, 2.0% or less.

In some embodiments, the sterile powder of the present disclosure contains less than 5% of prototype drug impurities after being placed at 25 °C and 60% RH for 30 days, and the content thereof may be less than 4.9%, 4.8%, 4.7%, 4.6%, 4.5%, 4.4%, 4.3%, 4.2%, 4.1%, 4.0%, 3.9%, 3.8%, 3.7%, 3.6%, 3.5%, 3.4%, 3.3%, 3.2%, 3.1%, 3.0%, 2.9%, 2.8%, 2.7%, 2.6%, 2.5%, 2.4%, 2.3%, 2.2%, 2.1%, 2.0% or less.

The present disclosure further provides a reconstituted solution obtained by reconstituting the sterile powder of the present disclosure.

In some embodiments, the reconstituted solution is an injectable composition.

The reconstituted solution may have a pH value of 3.0-8.0, preferably 5.0-8.0. The reconstituted solution may use water for injection, physiological saline, a glucose solution, etc.

The present disclosure further provides a method for preparing the pharmaceutical composition of the present disclosure, wherein the method comprises the step of mixing a compound of formula I or a pharmaceutically acceptable salt thereof with a stabilizer.

In some embodiments, the method further comprises the step of drying the composition comprising the compound of formula I or the pharmaceutically acceptable salt thereof and the stabilizer. The drying method may be selected from freeze-drying or spray-drying.

In some embodiments, the stabilizer is formulated in the form of a solution. Preferably, the stabilizer solution has a pH < 7.

The compound of formula I or the pharmaceutically acceptable salt thereof of the present disclosure may be a compound of formula I in any form, specific forms including, but not limited to, amorphous form, any crystal form, hydrate, solvate, etc.

The present disclosure further provides the use of the aforementioned pharmaceutical composition, sterile powder or reconstituted solution in the preparation of a drug for treating physiological disorders, conditions or diseases in a patient, wherein the physiological disorders, conditions or diseases are preferably respiratory diseases, cough, inflammatory diseases, skin disorders, ophthalmic disorders, depression, anxiety, phobia, bipolar disorder, alcohol dependence, neuroactive substance abuse, epilepsy, nociception, psychosis, schizophrenia, Alzheimer's disease, AIDS-related dementia, Towne's disease, stress-related disorders, obsessive/compulsive disorders, bulemia, anorexia nervosa, binge eating, mania, premenstrual syndrome, gastrointestinal dysfunction, atherosclerosis, fibrotic disorder, obesity, type II diabetes, headache, neuropathic pain, post-exercise pain, chronic pain syndrome, bladder disorders, urogenital disorders, vomiting or nausea.

The "mass volume percentage" (w/v) of the present disclosure refers to the mass (in g) of the component contained per 100 mL of a liquid system, i.e., g/100 mL.

The content (including percentage content) of various substances and the ratios of the various substances to each other of the present disclosure all have an allowable error of ± 5%. For example, the expression "the content of the compound of formula I or the pharmaceutically acceptable salt thereof in the composition is 5 to 20 mg/mL" means that the content of the compound of formula I or the pharmaceutically acceptable salt thereof in the composition is 4.75 to 21 mg/mL, which falls within the scope of the present disclosure; the expression "the weight ratio of the compound of formula I or the pharmaceutically acceptable salt thereof to the stabilizer is selected from 1 : 1 to 1 : 100" means that the weight ratio of the compound of formula I or the pharmaceutically acceptable salt thereof to the stabilizer is selected from 1 : 0.95 to 1 : 105, which falls within the scope of the present disclosure.

The expressions "mixing...into..." and "mixing... with..." of the present disclosure mean that the adding sequence of components is not limited. For example, "mixing A into B" can mean adding A to B, and can also mean adding B to A, and "mixing A with B" can mean adding A to B for mixing, and can also mean adding B to A for mixing.

### DETAILED DESCRIPTION OF THE PREFERRED EMBODIMENT

### Example 1

According to the ratio (w/v) in the table below, 90% of the target volume of a solvent was taken, a prescription amount of a stabilizer was added to the solvent, the pH of the resulting solution was adjusted with hydrochloric acid to pH < 7, to the solution was added the raw material of the compound of formula I, and the resulting mixture was stirred for dissolution at room temperature. The pH was adjusted with dilute hydrochloric acid or sodium hydroxide, filtration was performed with a 45 µm organic filtration membrane to obtain a clear solution, and the volume of the clear solution was made up to with the solvent. The above-mentioned solution was stored at 4 °C before subpackaging. After subpackaging according to the target volume, half of a stopper was pressed, and a freeze-drying procedure was run. After the completion of the procedure, nitrogen was filled, the remaining stopper was pressed, and the freeze-dried compounds were taken out of the box.

| No. | Content of compound of formula I | Stabilizer | Content of stabilizer | Solvent system | System pH | Other auxiliary materials |
|---|---|---|---|---|---|---|
| 1 | 2% | Sucrose | 5% | Water | 8 | |
| 2 | 2% | Glucose | 5% | Water | 8 | |
| 3 | 2% | Lactose | 2% | Water | 8 | |
| 4 | 2% | Arginine | 5% | Water | 8 | |
| 5 | 2% | Histidine | 2% | Water | 8 | |
| 6 | 2% | Proline | 5% | Water | 8 | |
| 7 | 2% | SBECD | 5% | Water | 8 | |
| 8 | 2% | Hydroxyethyl starch | 2% | Water | 8 | |
| 9 | 2% | PEG 4000 | 2% | Water | 8 | |
| 10 | 2% | PVP K12 | 2% | Water | 8 | |
| 11 | 2% | Dextran 40k | 2% | Water | 8 | |
| 12 | 4.36% | Trehalose | 5% | 50% TBA | 5 | |
| 13 | 4.36% | Mannitol | 5% | 50% TBA | 5 | |
| 14 | 4.35% | NADA | 8% | 50% TBA | 5 | |
| 15 | 3.00% | Maltose | 5% | 50% TBA | 5 | |
| 16 | 3.0% | Arginine | 5% | 50% TBA | 5 | 20 mM citrate buffer |
| 17 | 2.1% | Arginine | 5% | Water | 8 | 0.05% EDTA-2NaCa |
| 18 | 3.00% | Arginine | 5% | 50% TBA | 3 | |
| 19 | 3.00% | Arginine | 5% | 50% TBA | 4 | |
| 20 | 3.00% | Arginine | 5% | 50% TBA | 5 | |
| 21 | 3.00% | Arginine | 5% | 50% TBA | 6 | |
| 22 | 3.00% | Arginine | 5% | 50% TBA | 7 | |
| 23 | 3.00% | Arginine | 5% | 50% TBA | 8 | |
| 24 | 3.00% | Sucrose | 5% | 50% TBA | 5 | |
| 25 | 3.00% | Sucrose | 5% | 50% TBA | 8 | |
| 26 | 3.00% | Glucose | 3% | 50% TBA | 5 | |
| 27 | 3.00% | Glucose | 3% | 50% TBA | 8 | |

| | | | | | | |
|---|---|---|---|---|---|---|
| 50% TBA: 50% aqueous tert-butanol solution; SBECD: sulfobutylether-β-cyclodextrin; PVP: polyvinylpyrrolidone; NADA: N-acetyl-D-alanine | | | | | | |

### Example 2

The preparation samples prepared in example 1 were placed under accelerated conditions (25 °C, 60% RH), and were detected for the active substance and impurity content. Detection method: detection was performed by a high performance liquid chromatography system, wherein the chromatographic column was ACE Excel 3 SuperC18, 4.6 × 150 mm, 3 µm, the mobile phase A was 0.01 mol/L potassium dihydrogen phosphate solution-pH 7.0, the mobile phase B was acetonitrile, and the detection wavelength was 210 nm.

The detection results are as shown in the table below. The prototype drug impurity is rolapitant formed by the degradation of the compound of formula I.

| No. | Stabilizer | Stability condition | | Purity (%) | Prototype drug impurity (%) |
|---|---|---|---|---|---|
| 1 | Sucrose | 0 | 0d | 99.33 | 0.46' |
| | | 25 °C/60% RH | 14 d | 98.82 | 0.94 |
| | | | 30d | 98.54 | 1.24 |
| | | | 60 d | 98.19 | 1.63 |
| 2 | Glucose | 0 | 0d | 99.42 | 0.36 |
| | | 25 °C/60% RH | 14 d | 99.38 | 0.48 |
| | | | 30 d | 99.15 | 0.72 |
| | | | 60 d | 98.47 | 1.37 |
| | | | 90 d | 97.73 | 1.89 |
| 3 | Lactose | 0 | 0d | 98.4 | 1.04 |
| | | 25 °C/60% RH | 14 d | 95.38 | 3.16 |
| 4 | Arginine | 0 | 0d | 99.43 | 0.37 |
| | | 25 °C/60% RH | 14 d | 99.25 | 0.57 |
| | | | 30d | 99.14 | 0.69 |
| | | | 60 d | 98.92 | 0.89 |
| | | | 90 d | 98.83 | 0.98 |
| 5 | Histidine | 0 | 0d | 98.37 | 1.12 |
| | | 25 °C/60% RH | 14 d | 94.58 | 3.89 |
| | | | 90 d | 88.43 | 9.01 |
| 6 | Proline | 0 | 0d | 99.39 | 0.43 |
| | | 25 °C/60% RH | 14 d | 96.53 | 2.79 |
| | | | 90 d | 50.71 | 40.80 |
| 7 | SBECD | 0 | 0 d | 93.28 | 3.6 |
| | | 25 °C/60% RH | 14 d | 81.15 | 10.92 |
| | | | 90 d | 63.95 | 22.77 |
| 8 | Hydroxyethyl starch | 0 | 0 d | 91.90 | 3.83 |
| | | 25 °C/60% RH | 14 d | 82.069 | 9.55 |
| 9 | PEG 4000 | 0 | 0 d | 90.44 | 4.74 |
| | | 25 °C/60% RH | 14 d | 78.772 | 10.881 |
| 10 | PVP K12 | 0 | 0d | 88.20 | 5.81 |
| | | 25 °C/60% RH | 14 d | 86.121 | / |
| 11 | Dextran 40k | 0 | 0d | 92.79 | 3.59 |
| | | 25 °C/60% RH | 14 d | 82.914 | 9.168 |
| 12 | Trehalose | 0 | 0 d | 99.087 | 0.504 |
| | | 25 °C/60% RH | 7 d | 98.36 | 1.06 |
| | | | 14 d | 97.63 | 1.58 |
| 13 | Mannitol | 0 | 0 d | 98.949 | 0.494 |
| | | 25 °C/60% RH | 7 d | 98.09 | 1.03 |
| | | | 14 d | 97.19 | 1.56 |
| 14 | NADA | 0 | 0 d | 98.87 | 0.39 |
| | | 25 °C/60% RH | 7 d | 97.85 | 1.01 |
| | | | 14 d | 97.15 | 1.45 |
| | | | 30 d | 95.65 | 2.36 |
| 15 | Maltose | 0 | 0 d | 99.24 | 0.41 |
| | | 25 °C/60% RH | 7 d | 98.48 | 0.91 |
| | | | 14 d | 97.80 | 1.42 |
| | | | 30d | 96.95 | 2.03 |
| 16 | Arginine | 0 | 0d | 99.19 | 0.43 |
| | | 25 °C/60% RH | 7 d | 99.13 | 0.51 |
| | | | 14 d | 98.91 | 0.63 |
| | | | 30d | 98.72 | 0.74 |
| | | | 60 d | 98.34 | 0.97 |
| | | | 90 d | 98.12 | 1.21 |
| 17 | Arginine | 0 | 0 d | 99.53 | 0.38 |
| | | 25 °C/60% RH | 7 d | 98.88 | 0.90 |
| | | | 14 d | 98.81 | 0.95 |
| | | | 30d | 99.10 | 0.78 |
| | | | 90 d | 98.72 | 1.00 |
| 18 | Arginine | 0 | 0 d | 99.21 | 0.40 |
| | | 25 °C/60% RH | 07 d | 98.86 | 0.64 |
| | | | 14 d | 98.49 | 0.76 |
| | | | 30 d | 97.95 | 1.24 |
| | | | 60 d | 97.33 | 1.82 |
| | | | 90 d | 96.51 | 2.74 |
| 19 | Arginine | 0 | 0 d | 99.24 | 0.41 |
| | | 25 °C/60% RH | 07 d | 98.92 | 0.63 |
| | | | 14 d | 98.61 | 0.80 |
| | | | 30d | 98.31 | 1.01 |
| | | | 60 d | 97.72 | 1.44 |
| | | | 90 d | 97.10 | 1.99 |
| 20 | Arginine | 0 | 0 d | 99.24 | 0.40 |
| | | 25 °C/60% RH | 07 d | 98.94 | 0.60 |
| | | | 14 d | 98.71 | 0.75 |
| | | | 30d | 98.36 | 0.96 |
| | | | 60 d | 97.75 | 1.37 |
| | | | 90 d | 97.25 | 1.88 |
| 21 | Arginine | 0 | 0 d | 99.25 | 0.39 |
| | | 25 °C/60% RH | 07 d | 98.96 | 0.58 |
| | | | 14 d | 98.78 | 0.69 |
| | | | 30d | 98.31 | 1.02 |
| | | | 60 d | 97.86 | 1.36 |
| | | | 90 d | 97.84 | 1.4 |
| 22 | Arginine | 0 | 0 d | 99.27 | 0.37 |
| | | 25 °C/60% RH | 07 d | 98.92 | 0.61 |
| | | | 14 d | 98.75 | 0.88 |
| | | | 30d | 98.34 | 1.06 |
| | | | 60 d | 97.88 | 1.40 |
| | | | 90 d | 97.56 | 1.76 |
| 23 | Arginine | 0 | 0 d | 99.20 | 0.43 |
| | | 25 °C/60% RH | 07 d | 98.69 | 0.80 |
| | | | 14 d | 98.60 | 0.89 |
| | | | 30d | 98.32 | 1.11 |
| | | | 60 d | 97.58 | 1.64 |
| | | | 90 d | 97.24 | 2.02 |
| 24 | Sucrose | 0 | 0 d | 99.25 | 0.39 |
| | | 25 °C/60% RH | 07 d | 98.53 | 0.94 |
| | | | 14 d | 98.08 | 1.34 |
| | | | 30 d | 97.22 | 1.98 |
| 25 | Sucrose | 0 | 0 d | 98.74 | 0.69 |
| | | 25 °C/60% RH | 07 d | 97.58 | 1.53 |
| | | | 14 d | 96.61 | 2.18 |
| | | | 30 d | 95.91 | 2.76 |
| 26 | Glucose | 0 | 0 d | 99.24 | 0.41 |
| | | 25 °C/60% RH | 07 d | 98.97 | 0.62 |
| | | | 14 d | 98.63 | 0.90 |
| | | | 30d | 98.03 | 1.23 |
| | | | 60 d | 97.34 | 1.85 |
| | | | 90 d | 95.82 | 3.37 |
| 27 | Glucose | 0 | 0d | 98.95 | 0.58 |
| | | 25 °C/60% RH | 07 d | 97.95 | 1.26 |
| | | | 14 d | 97.93 | 1.31 |
| | | | 30 d | 97.35 | 1.70 |
| | | | 60 d | 97.40 | 1.87 |
| | | | 90 d | 96.17 | 2.71 |

After being placed under accelerated conditions, the freeze-dried preparations in which the stabilizers are sucrose, glucose and arginine have little change in purity, and are low in the prototype drug impurity from degradation, indicating good stability.

### Example 3

A compound freeze-dried preparation comprising the compound of formula I and palonosetron hydrochloride was prepared using the method of example 1 according to the ratio (w/v) in the table below.

| No. | Content of compound of formula I | Content of palonosetron hydrochloride | Stabilizer | Solvent system | System pH | Buffer | Chelating agent |
|---|---|---|---|---|---|---|---|
| 1 | 3.11% | 0.004011% | 5% Arginine | Water | 6.5 | 20 mM Citrate | 0.5% EDTA-2NaCa |

### Example 4

The preparation sample prepared in example 3 was placed under accelerated conditions (25 °C, 60% RH), and was detected for the active substance and impurity content. The results are as shown in the table below.

| No. | Stability condition | | Total impurity content (%) | Prototype drug impurity (%) |
|---|---|---|---|---|
| | 0 | 0 d | 1.18 | 0.37 |
| 1 | 25 °C/60% RH | 7 d | 1.32 | 0.53 |
| | | 1 M | 1.42 | 0.82 |
| | | 2 M | 1.48 | 1.07 |

## Claims

1. A pharmaceutical composition, comprising a compound of formula I or a pharmaceutically acceptable salt thereof and a stabilizer, wherein the stabilizer is an amino acid and/or a saccharide,

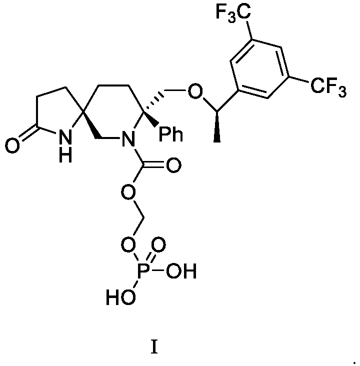

2. The pharmaceutical composition according to claim 1, wherein the amino acid is an amino acid that contains at least one carboxylic acid group and at least one primary amino group or secondary amino group but does not contain a secondary amide group, preferably alanine, 4-aminobutyric acid, 3-aminopentanoic acid, 5-aminopentanoic acid, 6-aminohexanoic acid, 8-aminooctanoic acid, arginine, aspartic acid, asparagine, cysteine, glutamic acid, glutamine, glycine, histidine, isoleucine, leucine, lysine, methionine, methyllysine, ornithine, phenylalanine, proline, serine, threonine, tryptophan, tyrosine and valine; or, the amino acid is a basic amino acid, preferably arginine, lysine and histidine, and more preferably arginine;
or, the saccharide is selected from one or more of sucrose, glucose, lactose, trehalose and maltose, preferably sucrose and/or glucose, and more preferably glucose;
or, the stabilizer is selected from one or more of arginine, glucose and sucrose, more preferably arginine and/or glucose.

3. The pharmaceutical composition according to any one of claims 1-2, wherein the pharmaceutical composition further comprises a solvent, preferably the solvent is selected from water, an organic solvent or a water/organic solvent cosolvent system, and more preferably the solvent is selected from water, ethanol, isopropanol, tert-butanol, water/ethanol, water/isopropanol or water/tert-butanol.

4. The pharmaceutical composition according to any one of claims 1-3, wherein the weight ratio of the compound of formula I or the pharmaceutically acceptable salt thereof to the stabilizer is 1 : 0.1-1 : 20, preferably 1 : 1-1 : 10, and more preferably 1 : 1-1 : 7.5.

5. The pharmaceutical composition according to claim 3, wherein the compound of formula I or the pharmaceutically acceptable salt thereof has a mass volume percentage of 0.01%-30%, preferably 0.01%-10%, and more preferably 0.1%-5%.

6. The pharmaceutical composition according to any one of claims 3-5, wherein the stabilizer has a mass volume percentage of 0.01%-30%, preferably 0.01%-20%, and more preferably 0.1%-10%.

7. The pharmaceutical composition according to any one of claims 1-6, wherein the pharmaceutical composition meets one or more of the following conditions:
(1) the pharmaceutical composition further comprises a buffer, the buffer is preferably a phosphate buffer, a citrate buffer, an acetate buffer, a succinate buffer, a histidine salt buffer or other organic acid salt buffers, and preferably the buffer has a concentration of 5 mM-40 mM, more preferably 10 mM-30 mM;
(2) the pharmaceutical composition further comprises a pH regulator;
(3) the pharmaceutical composition further comprises a chelating agent, preferably the chelating agent is ethylenediamine tetraacetic acid or a salt thereof, preferably the chelating agent has a mass volume percentage of 0.001%-5%, more preferably 0.01%-2%, and preferably the weight ratio of the compound of formula I or the pharmaceutically acceptable salt thereof to the chelating agent is 1000 : 1-1 : 1, more preferably 1000 : 1-10 : 1;
(4) the pharmaceutical composition has a pH value of 3.0-8.0, preferably 5.0-8.0;
(5) the pharmaceutical composition further comprises a 5-HT3 receptor antagonist, preferably the 5-HT3 receptor antagonist is selected from granisetron, ondansetron, ramosetron, tropisetron, palonosetron or dolasetron, more preferably palonosetron, preferably the 5-HT3 receptor antagonist has a mass volume percentage of 0.0001%-1%, more preferably 0.0001%-0.1%, and preferably the weight ratio of the compound of formula I or the pharmaceutically acceptable salt thereof to the 5-HT3 receptor antagonist is 2000 : 1-10 : 1, more preferably 1000 : 1-100 : 1.

8. The pharmaceutical composition according to any one of claims 1-7, comprising:
a compound of formula I or a pharmaceutically acceptable salt thereof;
a stabilizer selected from one or more of arginine, glucose and sucrose;
optionally palonosetron or a pharmaceutically acceptable salt thereof;
optionally a buffer;
optionally a chelating agent.

9. The pharmaceutical composition according to any one of claims 1-7, comprising:
0.01%-10% of a compound of formula I or a pharmaceutically acceptable salt thereof;
0.01%-20% of a stabilizer selected from one or more of arginine, glucose and sucrose;
optionally 0.0001%-1% of palonosetron or a pharmaceutically acceptable salt thereof;
optionally a 5 mM-40 mM buffer;
optionally 0.01%-2% of a chelating agent;
70%-99% of a solvent selected from water and/or tert-butanol;
the percentages are based on mass volume percentages.

10. A sterile powder obtained by freeze-drying or spray-drying the pharmaceutical composition according to any one of claims 1-9.

11. A sterile powder, which is reconstituted to obtain the pharmaceutical composition according to any one of claims 1-9.

12. The sterile powder according to claim 10 or 11, wherein the sterile powder meets one or two of the following conditions:
(1) the sterile powder contains less than 5% of prototype drug impurities, preferably less than 4%, more preferably less than 3%, and most preferably less than 2%.
(2) the sterile powder contains less than 5% of prototype drug impurities after being placed at 25 °C and 60% RH for 30 days.

13. A reconstituted solution obtained by reconstituting the sterile powder according to any one of claims 10-12.

14. A method for preparing the pharmaceutical composition according to any one of claims 1-9, wherein the method comprises the step of mixing a compound of formula I or a pharmaceutically acceptable salt thereof with a stabilizer.

15. The pharmaceutical composition according to any one of claims 1-9, the sterile powder according to any one of claims 10-12 or the reconstituted solution according to claim 13 for use in the treatment of physiological disorders, conditions or diseases in a patient, wherein the physiological disorders, conditions or diseases are preferably respiratory diseases, cough, inflammatory diseases, skin disorders, ophthalmic disorders, depression, anxiety, phobia, bipolar disorder, alcohol dependence, neuroactive substance abuse, epilepsy, nociception, psychosis, schizophrenia, Alzheimer's disease, AIDS-related dementia, Towne's disease, stress-related disorders, obsessive/compulsive disorders, bulemia, anorexia nervosa, binge eating, mania, premenstrual syndrome, gastrointestinal dysfunction, atherosclerosis, fibrotic disorder, obesity, type II diabetes, headache, neuropathic pain, post-exercise pain, chronic pain syndrome, bladder disorders, urogenital disorders, vomiting or nausea.

## Patentansprüche

1. Pharmazeutische Zusammensetzung, umfassend eine Verbindung der Formel I oder ein pharmazeutisch annehmbares Salz davon und einen Stabilisator, wobei der Stabilisator eine Aminosäure und/oder ein Saccharid ist:

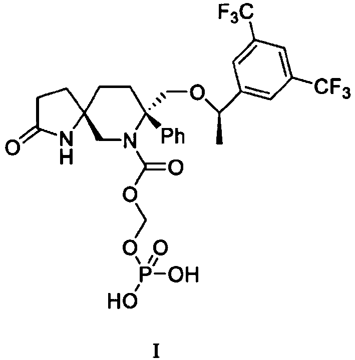

2. Pharmazeutische Zusammensetzung gemäß Anspruch 1, wobei die Aminosäure eine Aminosäure ist, die wenigstens eine Carbonsäuregruppe und wenigstens eine primäre Aminogruppe oder sekundäre Aminogruppe enthält, aber keine sekundäre Amidgruppe enthält, vorzugsweise Alanin, 4-Aminobuttersäure, 3-Aminopentansäure, 5-Aminopentansäure, 6-Aminohexansäure, 8-Aminooctansäure, Arginin, Asparaginsäure, Asparagin, Cystein, Glutaminsäure, Glutamin, Glycin, Histidin, Isoleucin, Leucin, Lysin, Methionin, Methyllysin, Ornithin, Phenylalanin, Prolin, Serin, Threonin, Tryptophan, Tyrosin und Valin; oder die Aminosäure eine basische Aminosäure ist, vorzugsweise Arginin, Lysin und Histidin, und besonders bevorzugt Arginin;
oder das Saccharid aus einem oder mehreren aus Saccharose, Glucose, Lactose, Trehalose und Maltose, vorzugsweise Saccharose und/oder Glucose und besonders bevorzugt Glucose ausgewählt ist;
oder der Stabilisator aus einem oder mehreren von Arginin, Glucose und Saccharose, besonders bevorzugt Arginin und/oder Glucose, ausgewählt ist.

3. Pharmazeutische Zusammensetzung gemäß einem der Ansprüche 1-2, wobei die pharmazeutische Zusammensetzung weiterhin ein Lösungsmittel umfasst, vorzugsweise das Lösungsmittel aus Wasser, einem organischen Lösungsmittel oder einem Cosolvenssystem aus Wasser/organischem Lösungsmittel ausgewählt ist, und besonders bevorzugt das Lösungsmittel aus Wasser, Ethanol, Isopropanol, tert-Butanol, Wasser/Ethanol, Wasser/Isopropanol oder Wasser/tert-Butanol ausgewählt ist.

4. Pharmazeutische Zusammensetzung gemäß einem der Ansprüche 1-3, wobei das Gewichtsverhältnis der Verbindung der Formel I oder ihres pharmazeutisch annehmbaren Salzes zu dem Stabilisator 1:0,1 bis 1:20, vorzugsweise 1:1 bis 1:10 und besonders bevorzugt 1:1 bis 1:7,5 beträgt.

5. Pharmazeutische Zusammensetzung gemäß Anspruch 3, wobei die Verbindung der Formel I oder ihr pharmazeutisch annehmbares Salz einen Massenanteil pro Volumen von 0,01% bis 30%, vorzugsweise 0,01% bis 10% und besonders bevorzugt 0,1% bis 5% aufweist.

6. Pharmazeutische Zusammensetzung gemäß einem der Ansprüche 3-5, wobei der Stabilisator einen Massenanteil pro Volumen von 0,01% bis 30%, vorzugsweise 0,01% bis 20% und besonders bevorzugt 0,1% bis 10% aufweist.

7. Pharmazeutische Zusammensetzung gemäß einem der Ansprüche 1-6, wobei die pharmazeutische Zusammensetzung eine oder mehrere der folgenden Bedingungen erfüllt:
(1) die pharmazeutische Zusammensetzung umfasst weiterhin einen Puffer, wobei es sich bei dem Puffer vorzugsweise um einen Phosphatpuffer, einen Citratpuffer, einen Acetatpuffer, einen Succinatpuffer, einen Histidinsalzpuffer oder andere Puffer aus Salzen organischer Säuren handelt und vorzugsweise weist der Puffer eine Konzentration von 5 mM-40 mM, besonders bevorzugt 10 mM-30 mM, auf;
(2) die pharmazeutische Zusammensetzung umfasst weiterhin einen pH-Regulator;
(3) die pharmazeutische Zusammensetzung umfasst weiterhin einen Chelatbildner, vorzugsweise handelt es sich bei dem Chelatbildner um Ethylendiamintetraessigsäure oder ein Salz davon, vorzugsweise hat der Chelatbildner einen Massenanteil pro Volumen von 0,001% bis 5%, besonders bevorzugt 0,01% bis 2%, und vorzugsweise beträgt das Gewichtsverhältnis der Verbindung der Formel I oder ihres pharmazeutisch annehmbaren Salzes zu dem Chelatbildner 1000:1 bis 1:1, besonders bevorzugt 1000:1 bis 10:1;
(4) die pharmazeutische Zusammensetzung hat einen pH-Wert von 3,0-8,0, vorzugsweise 5,0-8,0;
(5) die pharmazeutische Zusammensetzung umfasst weiterhin einen 5-HT3-Rezeptor-Antagonisten, vorzugsweise ist der 5-HT3-Rezeptor-Antagonist aus Granisetron, Ondansetron, Ramosetron, Tropisetron, Palonosetron oder Dolasetron, besonders bevorzugt Palonosetron, ausgewählt, vorzugsweise hat der 5-HT3-Rezeptor-Antagonist einen Massenanteil pro Volumen von 0,0001% bis 1%, besonders bevorzugt 0,0001% bis 0,1%, und vorzugsweise beträgt das Gewichtsverhältnis der Verbindung der Formel I oder ihres pharmazeutisch annehmbaren Salzes zu dem 5-HT3-Rezeptor-Antagonist 2000:1 bis 10:1, besonders bevorzugt 1000:1 bis 100:1.

8. Pharmazeutische Zusammensetzung gemäß einem der Ansprüche 1-7, umfassend:
eine Verbindung der Formel I oder ein pharmazeutisch annehmbares Salz davon;
einen Stabilisator, der aus einem oder mehreren von Arginin, Glucose und Saccharose ausgewählt ist;
gegebenenfalls Palonosetron oder ein pharmazeutisch annehmbares Salz davon;
gegebenenfalls einen Puffer;
gegebenenfalls einen Chelatbildner.

9. Pharmazeutische Zusammensetzung gemäß einem der Ansprüche 1-7, umfassend:
0,01%-10% einer Verbindung der Formel I oder eines pharmazeutisch annehmbaren Salzes davon;
0,01%-20% eines Stabilisators, der aus einem oder mehreren von Arginin, Glucose und Saccharose ausgewählt ist;
gegebenenfalls 0,0001%-1% Palonosetron oder eines pharmazeutisch annehmbaren Salzes davon;
gegebenenfalls einen 5 mM bis 40 mM Puffer;
gegebenenfalls 0,01%-2% eines Chelatbildners;
70%-99% eines Lösungsmittels, das aus Wasser und/oder tert-Butanol ausgewählt ist;
wobei die Prozentangaben auf Massenprozentanteile pro Volumen bezogen sind.

10. Steriles Pulver, erhalten durch Gefriertrocknen oder Sprühtrocknen der pharmazeutischen Zusammensetzung gemäß einem der Ansprüche 1-9.

11. Steriles Pulver, das rekonstituiert wird, wobei man die pharmazeutische Zusammensetzung gemäß einem der Ansprüche 1-9 erhält.

12. Steriles Pulver gemäß Anspruch 10 oder 11, wobei das sterile Pulver eine oder zwei der folgenden Bedingungen erfüllt:
(1) das sterile Pulver enthält weniger als 5% Verunreinigungen von Arzneimittel-Prototypen, vorzugsweise weniger als 4%, besonders bevorzugt weniger als 3% und am meisten bevorzugt weniger als 2%;
(2) das sterile Pulver enthält weniger als 5% Verunreinigungen von Arzneimittel-Prototypen, nachdem es 30 Tage lang bei 25°C und 60% relativer Feuchtigkeit aufbewahrt wurde.

13. Rekonstituierte Lösung, erhalten durch Rekonstituieren des sterilen Pulvers gemäß einem der Ansprüche 10-12.

14. Verfahren zur Herstellung der pharmazeutischen Zusammensetzung gemäß einem der Ansprüche 1-9, wobei das Verfahren den Schritt des Mischens einer Verbindung der Formel I oder eines pharmazeutisch annehmbaren Salzes davon mit einem Stabilisator umfasst.

15. Pharmazeutische Zusammensetzung gemäß einem der Ansprüche 1-9, steriles Pulver gemäß einem der Ansprüche 10-12 oder rekonstituierte Lösung gemäß Anspruch 13 zur Verwendung bei der Behandlung von physiologischen Störungen, Zuständen oder Erkrankungen bei einem Patienten, wobei es sich bei den physiologischen Störungen, Zuständen oder Erkrankungen vorzugsweise um Atemwegserkrankungen, Husten, Entzündungskrankheiten, Hautstörungen, Augenstörungen, Depressionen, Angstzustände, Phobien, bipolare Störung, Alkoholabhängigkeit, Missbrauch neuroaktiver Substanzen, Epilepsie, Nozizeption, Psychose, Schizophrenie, Alzheimer-Krankheit, AIDS-bedingte Demenz, Frühdemenz, stressbedingte Störungen, Zwangsneurosen, Bulimie, Anorexia nervosa, Binge-Eating-Störung, Manie, prämenstruelles Syndrom, gastrointestinale Dysfunktion, Atherosklerose, fibrotische Störung, Adipositas, Typ-II-Diabetes, Kopfschmerzen, neuropathische Schmerzen, Muskelkater, chronisches Schmerzsyndrom, Blasenstörungen, urogenitale Störungen, Erbrechen oder Übelkeit handelt.

## Revendications

1. Composition pharmaceutique, comprenant un composé répondant à la formule I ou un sel pharmaceutiquement acceptable de celui-ci, et un stabilisateur, dans laquelle le stabilisateur est un acide aminé et/ou un saccharide,

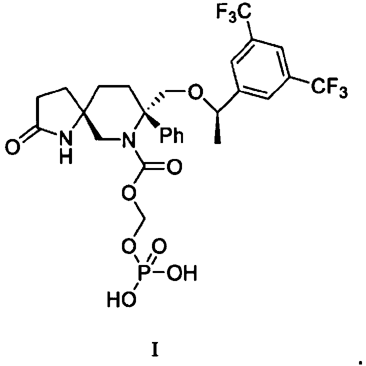

2. Composition pharmaceutique selon la revendication 1, dans laquelle l'acide aminé est un acide aminé qui contient au moins un groupe d'acide carboxylique et au moins un groupe aminé primaire ou groupe aminé secondaire, mais ne contient pas de groupe amide secondaire, de préférence alanine, acide 4-aminobutyrique, acide 3-aminopentanoïque, acide 5-aminopentanoïque, acide 6-aminohexanoïque, acide 8-aminooctanoïque, arginine, acide aspartique, asparagine, cystéine, acide glutamique, glutamine, glycine, histidine, isoleucine, leucine, lysine, méthionine, méthyllysine, ornithine, phénylalanine, proline, sérine, thréonine, tryptophane, tyrosine et valine ; ou l'acide aminé est un acide aminé basique, de préférence arginine, lysine et histidine, et de préférence encore arginine ;
ou le saccharide est choisi parmi l'un ou plusieurs parmi saccharose, glucose, lactose, tréhalose et maltose, de préférence saccharose et/ou glucose, et de préférence encore glucose ;
ou le stabilisateur est choisi parmi l'un ou plusieurs parmi arginine, glucose et saccharose, de préférence encore arginine et/ou glucose.

3. Composition pharmaceutique selon l'une quelconque des revendications 1-2, dans laquelle la composition pharmaceutique comprend en outre un solvant, de préférence le solvant est choisi parmi l'eau, un solvant organique ou un système de cosolvants eau/solvant organique, et de préférence encore le solvant est choisi parmi eau, éthanol, isopropanol, tert-butanol, eau/éthanol, eau/isopropanol ou eau/tert-butanol.

4. Composition pharmaceutique selon l'une quelconque des revendications 1-3, dans laquelle le rapport pondéral du composé répondant à la formule I ou son sel pharmaceutiquement acceptable au stabilisateur est de 1 : 0,1 à 1 : 20, de préférence 1 : 1 à 1 : 10, et de préférence encore 1 : 1 à 1 : 7,5.

5. Composition pharmaceutique selon la revendication 3, dans laquelle le composé répondant à la formule I ou son sel pharmaceutiquement acceptable présente un pourcentage massique volumique de 0,01 % à 30 %, de préférence 0,01 % à 10 %, et de préférence encore 0,1 % à 5 %.

6. Composition pharmaceutique selon l'une quelconque des revendications 3-5, dans laquelle le stabilisateur présente un pourcentage massique volumique de 0,01 % à 30 %, de préférence 0,01 % à 20 %, et de préférence encore 0,1 % à 10 %.

7. Composition pharmaceutique selon l'une quelconque des revendications 1-6, dans laquelle la composition pharmaceutique satisfait une ou plusieurs des conditions suivantes :
(1) la composition pharmaceutique comprend en outre un tampon, le tampon est de préférence un tampon phosphate, un tampon citrate, un tampon acétate, un tampon succinate, un tampon sel d'histidine ou d'autres tampons sels d'acides organiques, et de préférence le tampon présente une concentration de 5 mM à 40 mM, de préférence encore 10 mM à 30 mM ;
(2) la composition pharmaceutique comprend en outre un régulateur de pH ;
(3) la composition pharmaceutique comprend en outre un agent chélateur, de préférence l'agent chélateur est l'acide éthylènediaminetétraacétique ou un sel de celui-ci, de préférence l'agent chélateur présente un pourcentage massique volumique de 0,001 % à 5 %, de préférence 0,01 % à 2 %, et de préférence le rapport pondéral du composé répondant à la formule I ou son sel pharmaceutiquement acceptable à l'agent chélateur est de 1000 : 1 à 1 : 1, de préférence encore 1000 : 1 à 10 : 1 ;
(4) la composition pharmaceutique présente une valeur pH de 3,0 à 8,0, de préférence 5,0 à 8,0 ;
(5) la composition pharmaceutique comprend en outre un antagoniste du récepteur 5-HT3, de préférence l'antagoniste du récepteur 5-HT3 est choisi parmi granisétron, ondansétron, ramosétron, tropisétron, palonosétron ou dolasétron, de préférence encore palonosétron, de préférence l'antagoniste du récepteur 5-HT3 présente un pourcentage massique volumique de 0,0001 % à 1 %, de préférence 0,0001 % à 0,1 %, et de préférence le rapport pondéral du composé répondant à la formule I ou son sel pharmaceutiquement acceptable à l'antagoniste du récepteur 5-HT3 est de 2000 : 1 à 10 : 1, de préférence encore 1000 : 1 à 100 : 1.

8. Composition pharmaceutique selon l'une quelconque des revendications 1-7, comprenant :
un composé répondant à la formule I ou un sel pharmaceutiquement acceptable de celui-ci ;
un stabilisateur choisi parmi l'un ou plusieurs parmi arginine, glucose et saccharose ;
éventuellement palonosétron ou un sel pharmaceutiquement acceptable de celui-ci ;
éventuellement un tampon ;
éventuellement un agent chélateur.

9. Composition pharmaceutique selon l'une quelconque des revendications 1-7, comprenant :
0,01 % à 10 % d'un composé répondant à la formule I ou d'un sel pharmaceutiquement acceptable de celui-ci ;
0,01 % à 20 % d'un stabilisateur choisi parmi l'un ou plusieurs parmi arginine, glucose et saccharose ;
éventuellement 0,0001 % à 1 % de palonosétron ou d'un sel pharmaceutiquement acceptable de celui-ci ;
éventuellement un tampon de 5 mM à 40 mM ;
éventuellement 0,01 % à 2 % d'un agent chélateur ;
70 % à 99 % d'un solvant choisi parmi l'eau et/ou le tert-butanol ;
les pourcentages étant des pourcentages massiques volumiques.

10. Poudre stérile, obtenue par lyophilisation ou séchage par atomisation de la composition pharmaceutique selon l'une quelconque des revendications 1-9.

11. Poudre stérile, qui est reconstituée pour obtenir la composition pharmaceutique selon l'une quelconque des revendications 1-9.

12. Poudre stérile selon la revendication 10 ou 11, dans laquelle ladite poudre stérile satisfait une ou deux des conditions suivantes :
(1) la poudre stérile contient moins de 5 % d'impuretés du médicament prototype, de préférence moins de 4 %, de préférence encore moins de 3 %, et de préférence maximale moins de 2 % ;
(2) la poudre stérile contient moins de 5 % d'impuretés du médicament prototype après avoir été placée à 25 °C et 60 % d'humidité relative pendant 30 jours.

13. Solution reconstituée, obtenue par reconstitution de la poudre stérile selon l'une quelconque des revendications 10-12.

14. Procédé pour préparer la composition pharmaceutique selon l'une quelconque des revendications 1-9, dans lequel le procédé comprend l'étape consistant à mélanger un composé répondant à la formule I ou un sel pharmaceutiquement acceptable de celui-ci avec un stabilisateur.

15. Composition pharmaceutique selon l'une quelconque des revendications 1-9, poudre stérile selon l'une quelconque des revendications 10-12 ou solution reconstituée selon la revendication 13 à utiliser dans le traitement des troubles, affections ou maladies physiologiques chez un patient, dans laquelle les troubles, affections ou maladies physiologiques sont de préférence des maladies respiratoires, toux, maladies inflammatoires, affections cutanées, troubles ophtalmiques, dépression, anxiété, phobie, trouble bipolaire, dépendance à l'alcool, abus de substances neuroactives, épilepsie, nociception, psychose, schizophrénie, maladie d'Alzheimer, démence liée au SIDA, démence précoce, troubles liés au stress, troubles obsessionnels-compulsifs, boulimie, anorexie mentale, hyperphagie boulimique, manie, syndrome prémenstruel, dysfonctionnement gastro-intestinal, athérosclérose, fibrose, obésité, diabète de type II, maux de tête, douleurs neuropathiques, douleurs post-exercice, syndrome de douleur chronique, troubles de la vessie, troubles urogénitaux, vomissements ou nausées.
